# EUROPEAN PATENT SPECIFICATION

(11) **EP 2 248 647 B1**
(45) Date of publication and mention of the grant of the patent: **10.12.2014**
(21) Application number: 09713126.2
(22) Date of filing: 13.02.2009
(51) Int. Cl.: B29C 39/02, A61F 2/16, A61L 27/00, B29L 11/00, B29D 11/00, B29D 11/02, B29L 31/00, B29C 35/08, G02B 1/04, B29C 39/26, B29C 39/00

(54) **METHOD OF PRODUCING INTRAOCULAR LENS**
VERFAHREN ZUR HERSTELLUNG EINER INTRAOKULARLINSE
PROCÉDÉ DE PRODUCTION DE LENTILLE INTRAOCULAIRE

(30) Priority: 20.02.2008 JP 2008038346
(43) Date of publication of application: 10.11.2010
(73) Proprietor: Hoya Corporation, Tokyo 161-8525 (JP)
(72) Inventor: SHOJI, Noriyuki, Tokyo 161-8525 (JP); INOUE, Masanobu, Tokyo 161-8525 (JP); SUZUKI, Hirofumi, Tokyo 161-8525 (JP)
(74) Representative: MERH-IP Matias Erny Reichl Hoffmann
(86) International application number: PCT/JP2009/052369
(87) International publication number: WO 2009/104516

(56) References cited:
- JP-A- 3 284 258
- JP-A- 4 275 110
- JP-A- 6 190 942
- JP-A- 60 073 836
- JP-A- 63 061 225
- JP-A- 63 068 668
- JP-A- 63 091 230
- JP-A- 2000 005 203
- JP-A- 2005 329 712
- JP-A- 2007 307 771
- US-A- 5 137 441
- US-A- 5 169 569
- US-A- 5 397 511
- US-A- 5 895 610
- US-B1- 6 555 030

## Description

### Technical Field

The present invention relates to a manufacturing method according to claim 1 of an intraocular lens for manufacturing an intraocular lens inserted into an eye instead of a crystalline lens of an eyeball.

### Background Art

When a crystalline lens is getting cloudy due to a cataract, etc, the crystalline lens is removed and an intraocular lens is inserted instead. In recent years, with a spread of an Ultrasonic Phacoemulsification and Aspiration in particular, an intraocular lens that can be inserted from a small incisional wound has been developed and is put to clinical use widely, for the purpose of reducing a postoperative astigmatism and a surgical invasion. This is a soft intraocular lens capable of inserting the intraocular lens from the small incisional wound with its optical part bent, by using a soft material in an optical part member.

From a structural aspect, the soft intraocular lens is classified broadly into a type that an optical part and a support part are composed of different kind of materials, and a type that the optical part and the support part are composed of the same material. The intraocular lens of the type that the optical part and the support part are composed of the different kind of material, is generally constituted of an approximately a circular optical part composed of a soft material such as foldable silicon, acrylic resin, and hydrogel, and a support part composed of relatively harder material such as polypropylene and polymethyl methacrylate, with its tip end opened. However, such a type of intraocular lens has a disadvantageous factor that a higher cost is required due to a complicated manufacturing step although stability in an eye is relatively satisfactory, and there is a high possibility that some trouble occurs at a joint part because the optical part and the support part are composed of the different kind of materials. Meanwhile, the soft intraocular lens with the optical part and the support part composed of the same material, does not have such a disadvantageous factor, and therefore has been highlighted recently.

Incidentally, as a manufacturing method of the soft intraocular lens with the optical part and the support part composed of the same material, a race cutting method and a cast molding method can be given. The race cutting method includes the steps of: applying mechanical machining process to an intraocular lens material including an optical surface; and molding it into a desired shape. Meanwhile, the cast molding method includes the steps of: injecting a raw material monomer into a mold having a shape of an intraocular lens; and polymerizing and molding the monomer. As a method utilizing the cast molding method, for example, methods disclosed in patent document 1 and patent document 2 are known.

According to the method disclosed in the patent document 1, one surface of a lens optical surface is formed by the cast molding method, and the other surface is formed by the machining process. According to the method disclosed in the patent document 2, both surfaces of the lens optical surface are formed by the cast molding method, and the support part is shaped by punching processing, laser processing, and cutting, etc.

A similiar method to patent document 2 is described in patent document 3.
Patent document 1 : Japanese Patent Laid Open Publication No.1988-91230
Patent document 2: Japanese Patent Laid Open Publication No.1992-284258
Patent document 3: United Staes Patent No. US 6 555 030

### Disclosure of the Invention

### Problem to be solved by the Invention

However, according to the aforementioned race cutting method, the optical surface of the intraocular lens composed of the soft material needs to be formed by mechanical cutting (race cutting), and for this purpose, a workpiece is cooled and hardened to a glass transition temperature or less. This involves a complicated step and makes it difficult to manage the workpiece, and further requires polishing for removing cutting marks. In recent years, lenses such as an aspherical lens and a refractive lens requiring extremely high degree of optical design have been developed. However, when such a lens requires polishing, the optical surface formed with high accuracy is destroyed by polishing, and generally it becomes difficult to exhibit a desired optical performance. Further, in a case that powders generated during processing are stuck to the optical part, such a cutting powder is hardly removed even by tumbling polishing which is frequently used in manufacturing IOL.

Meanwhile, the method utilizing the aforementioned cast molding is preferable in a point that there is no problem given in the aforementioned race cutting method. However, in a case of the cast molding method, particularly in a case of a loop type in which the support part is narrow and thin, there is a problem that deformation occurs due to contraction during polymerization of the monomer, or a problem that a desired shape and size of a lens are hardly obtained. Further, even when the cast molding method is utilized, the cutting process is needed in most cases in a process of a final product. In this case, there is also a problem that the cutting powder is stuck to the optical part as described above. In order to solve the above-described problems, the present invention is provided, and an object of the present invention is to provide an intraocular lens manufacturing method capable of eliminating a possibility of sticking of the cutting powder or dust generated during processing, and capable of stably manufacturing the intraocular lens having a desired accuracy at a relatively low cost.

### Means for solving the problem

First means for solving the above-described problems is a manufacturing method of an intraocular lens comprising an optical part having a front optical surface and a rear optical surface that constitute front and rear surfaces of a lens; and a support part provided on an outer peripheral part of the optical part for supporting the optical part in an eye,
wherein a mold is used, having an optical part molding part for forming a member becoming the optical part by molding, and a support part molding part for forming a member becoming the support part by molding, wherein the front optical surface and the rear optical surface can be directly obtained by molding the optical molding part, and the support part can be obtained by applying a additional machining to the member obtained by molding,
the method comprising the steps of:
injecting a raw material of an intraocular lens into the mold, then polymerizing or hardening the raw material, then
machining the member becoming the support part into a shape of the support part in a state that at least the front optical surface and the rear optical surface in the polymerized or hardened members are covered with the optical part molding part of the mold, and
releasing the optical part molding part from the mold.
Second means is the manufacturing method of the intraocular lens according to the first means,
wherein the support part molding part of the mold has a sectional face with a shape of a completed support part if cut by a sectional face including an optical axis of the optical part, and with an arbitrary shape if viewed from the optical axis direction, and machining by the support part molding part is applied to the member becoming the support part so that the shape of the member becoming the support part viewed from the optical axis direction corresponds to the shape of the completed support part.

Third means is the manufacturing method of the intraocular lens according to the first or the second means, wherein a raw material of the intraocular lens is injected into the mold, which is then polymerized or hardened, and in a state that polymerized or hardened members exist in both the optical part molding part and the support part molding part of the mold, the members are machined together with the mold so that the shape viewed from the optical axis direction in the member becoming the optical part and the member becoming the support part corresponds to the shape of the completed intraocular lens.

Fourth means is the manufacturing method of the intraocular lens according to any one of the first to third means, wherein the mold has an upper mold and a lower mold,
the method comprising the steps of:
injecting the raw material of the intraocular lens into the mold, then polymerizing or hardening the raw material; then
machining the member becoming the optical part and/or the member becoming the support part together with the mold in a state that the members exist between the upper mold and the lower mold, so that the shape viewed from the optical axis direction corresponds to a completed shape,
wherein in the machining step, machining is applied to the mold with the members existing inside in such a manner as digging into one of the upper mold and the lower mold of the mold from the optical axis direction along a virtual contour line that partitions so that the shape viewed from the optical axis direction corresponds to the completed shape to a depth of passing through one of the molds and to a prescribed depth of the other mold, over an entire circumference of the contour line.

Fifth means is the manufacturing method of the intraocular lens according to any one of the first to fourth means, wherein the intraocular lens is a soft intraocular lens.

Sixth means is the manufacturing method of the intraocular lens according to any one of the first to fifth means, wherein the mold is composed of plastic resin.

Seventh means is the manufacturing method of the intraocular lens according to the sixth means, wherein the plastic resin is polyolefin resin, polyethylene resin, or polypropylene resin.

Eighth means is the manufacturing method of the intraocular lens according to the sixth means, wherein a glass transition temperature of a material composing the soft intraocular lens is less than 35°C.

Ninth means is the manufacturing method of the intraocular lens according to the sixth means, wherein a material composing the soft intraocular lens is a polymer of acrylic monomer.

Tenth means is the manufacturing method of the intraocular lens according to the sixth means, wherein a treatment for imparting adhesive strength to the material composing the optical part is previously applied to an inner surface of the mold made of plastic resin.

Eleventh means is the manufacturing method of the intraocular lens according to the tenth means, wherein in the treatment for imparting adhesive strength to the material, an active light, which has its emission peak at 150-300 nm wavelength range and has a function that decomposes an oxygen molecule to generate ozone and decomposes the ozone to generate active oxygen species, is previously irradiated to the first polymer under existence of oxygen.

### Advantage of the Invention

According to the above-described means, an intraocular lens can be manufactured, by which the possibility that cutting powder or dust generated during machining can be eliminated and in addition, the intraocular lens having a desired accuracy can be stably manufactured at a relatively low cost, by selecting a material.

### Best Mode for carrying out the Invention

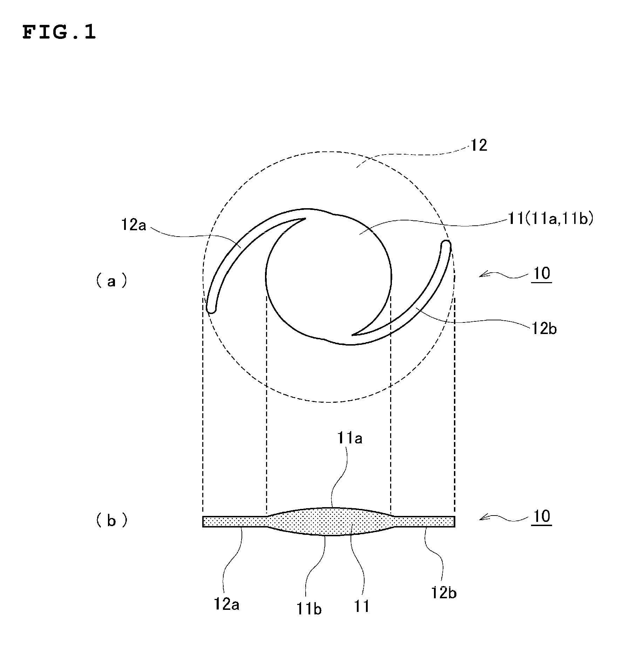

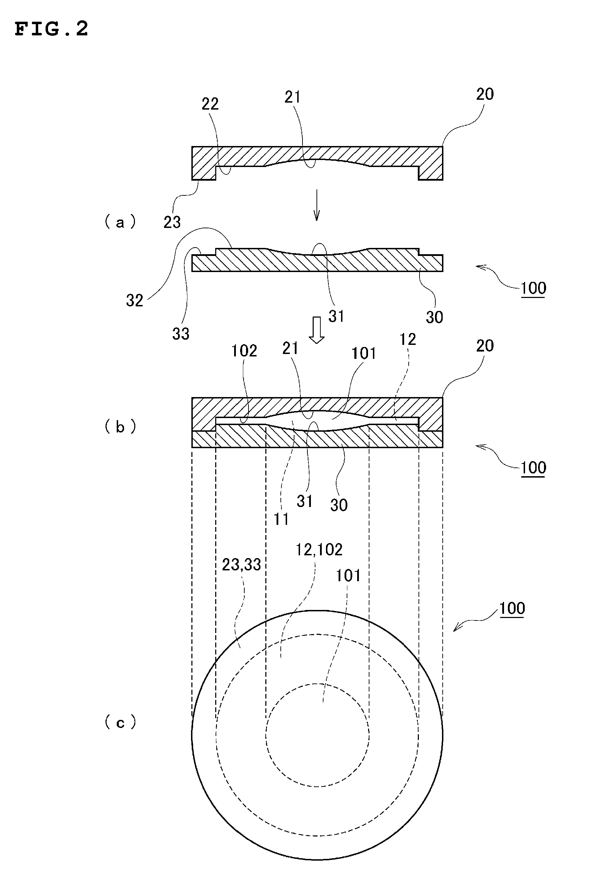

FIG. 1 is a view showing an intraocular lens manufactured by a manufacturing method of the intraocular lens according to an embodiment of the present invention, wherein FIG. 1(a) is a plan view, FIG. 1(b) is a side view. FIG. 2 is a view showing a mold used for the manufacturing method of the intraocular lens according to an embodiment of the present invention, wherein FIG. 2(a) is a cross-sectional view showing a state that an upper mold and a lower mold are separated from each other, and FIG. 2 (b) is a cross-sectional view showing a state that the upper mold and the lower mold are connected to each other in a state of being molded, and FIG. 2 (c) is a plan view. First, the intraocular lens manufactured by the manufacturing method of the intraocular lens according to the embodiment of the present invention and the mold used for the manufacturing method of the intraocular lens according to the embodiment of the present invention will be described hereafter, with reference to FIG. 1 and FIG. 2.

As shown in FIG. 1, an intraocular lens 10 manufactured by the manufacturing method of the intraocular lens according to the embodiment of the present invention has an optical part 11, and two support parts 12a and 12b provided on an outer peripheral part of the optical part 11 so as to support the optical part 11 in an eye. The optical part 11 has a front optical surface 11a and a rear optical surface 11b constituting front and rear surfaces of a lens. Note that the intraocular lens 10 is obtained by molding an approximately a discoid molded product in which the optical part 11 is formed in a central part and a support part precursor member 12 (a part shown by dot line in FIG. 1(a)), being a member formed in an annular shape and a member becoming a support part is formed in its peripheral part, and machining the support part precursor member 12 in this molded product into shapes of the support parts 12a and 12b by machining such as cutting.

Further, as shown in FIG. 2, a mold 100 used for manufacturing the intraocular lens according to the embodiment of the present invention has an upper mold 20 and a lower mold 30. As shown in FIG. 2 (a), the upper mold 20 has a front optical surface molding part 21 for forming a front optical surface 11a in the optical part 11 of the intraocular lens 10, a support part molding part 22 for molding members becoming the support parts 12a and 12b, and a flange part 23, being a member for partitioning these molding parts. The lower mold 30 has a rear optical surface molding part 31 for forming a rear optical surface 11b in the optical part 11 of the intraocular lens 10, a support part molding part 32 for molding members becoming the support parts 12a and 12b, and a flange receiving part 33 for receiving the flange part 23 of the upper mold 20. Further, monomer reservoir or air ventilation may also be provided, which is ordinarily used in a cast molding method.

As shown in FIG. 2(b), when the upper mold 20 and the lower mold 30 are connected to each other, so that the flange part 23 of the upper mold 20 is fitted into a flange receiving part 32 of the lower mold 30 in a state of molding the upper mold 20 and the lower mold 30, spaces are formed between the upper mold 20 and the lower mold 30. Then, among such spaces, a space formed in the central part becomes an optical molding part 101, and a space formed in its periphery becomes a support part molding part 102. Thus, for example, the approximately discoid molding product can be molded, in which the optical part 11 is formed in the central part and the annular support part precursor member 12 is formed in its peripheral part, by supplying the raw material to the lower mold 30 for molding, and connecting the upper mold 22 to the lower mold 30.

A cuttable plastic resin mold is used as the upper mold 20 and the lower mold 30. However, it is preferable to use plastic resin having excellent solvent resistance which does not generate deformation of a lens material due to a raw material monomer, and particularly a polymerization container made of polyolefin resin such as polyethylene and polypropylene is preferable. Further, adhesive strength imparting treatment for imparting adhesive strength to the lens material polymerized in the mold may be previously applied to an inner wall of the plastic resin mold. Owing to appropriate adhesive strength imparting treatment, separation between the lens material obtained after polymerization and the mold can be prevented, and a reliable transfer of the inner surface shape of the mold is realized, and in addition adhesive strength between the lens material and the mold is enhanced and peeling off and protrusion of the lens material from the mold during machining can be prevented. The adhesive strength imparting treatment is not particularly limited, provided that the adhesion to the polymerized lens material is enhanced, and for example, a conventionally known technique and apparatus such as frame treatment, primer treatment, ultraviolet radiation treatment, and electrical surface treatment, etc, can be used. As the shape of the optical surface (front optical surface 11a, rear optical surface 11b) provided on the inner surface of the mold, spherical, aspherical, toric, bifocal, multifocal, and further refractive, diffractive, etc, surfaces can be given. However, the shape of the optical surface is not particularly limited, provided that it is effective as an optical design of the intraocular lens, and also an outer shape of the mold is not particularly limited, provide that it is a suitable shape for manufacture.

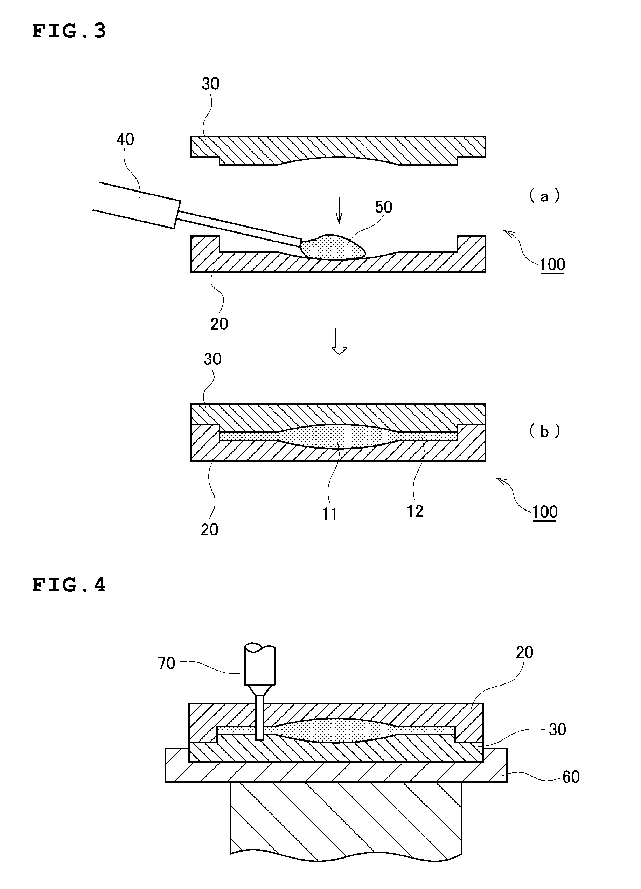

FIG. 3 to FIG. 9 are views for explaining the manufacturing method of the intraocular lens according to embodiments of the present invention. The manufacturing method of the intraocular lens according to the embodiment of the present invention will be described hereafter, with reference to the drawings.

FIG. 3 is a view showing a state of injecting and molding the raw material into the mold 100. As shown in FIG. 3, raw material monomer 50 of the lens material are injected into the upper mold 20 by a raw material injector 40 (see FIG. 3(a)), then the lower mold 30 is assembled thereinto and a lid is put thereon (see FIG. 3(b)). The raw material monomer of the lens material used here is not particularly limited, provide that it is publicly-known monomer, and publicly-known thermal polymerization initiator and photopolymerization initiator, etc, can be used as polymerization initiators. Further, in order to give a UV absorbing power to the obtained intraocular lens or coloring it, for example, a polymerizing ultraviolet ray absorber and a polymerizing pigment, etc, can be used as copolymer components.

Next, the raw material monomer molded by the upper mold 20 and the lower mold 30 is polymerized in these molds. As a method of polymerization, for example, a method of heating and polymerizing the raw material monomer can be given, wherein a temperature is increased gradually or continuously in a temperature range of 25 to 120°C and polymerization is completed in several hours to several tens of hours. Further, for example, a method of performing photopolymerization can be given, wherein polylmerization is performed by irradiating the monomer with light beam with a wave length determined in accordance with absorption of activity of a photo initiator such as ultraviolet ray or visible light. Further, for example, there is a method of performing polymerization in combination of the method of heating and polymerizing the monomer and the method of performing photopolymerization. At this time, atmosphere in a tank or a chamber for polymerization is set in the atmosphere of inert gas such as argon. Atmospheric air or pressurized state may also be allowed for polymerization. Thus, a discoid polymer 12 is formed, having the optical part 11 in the central part with the front optical surface 11a and the rear optical surface 11b formed therein, and having the support part precursor material 12 formed in its circumference.

Next, the discoid polymer 12 thus obtained is subjected to machining into a lens shape by mechanically cutting process, by not taking out from the mold 100 but in a state of being pinched between the upper mold 20 and the lower mold 30, namely together with the mold 100 (see FIG. 4 to FIG. 6). Here, FIG. 4 is a cross-sectional view showing a state that the discoid polymer 12 is fixed to a fixing jig 60 and is machined by an endmill 70 together with the mold 100, FIG. 5 is a perspective view showing a state that the discoid polymer 12 is subjected to machining by the endmill 70 together with the mold 100, and FIG. 6 is a view showing a state that the discoid polymer 12 is subjected to machining by the endmill 70 together with the mold 100 wherein FIG. 6(a) is a cross-sectional view and FIG. 6(b) is a plan view.

As shown in FIG. 4, during machining, the endmill 70 that machines the lens shape digs into the lower mold 30 in a machining direction at a depth of slightly forming a slit thereon, so as to pass through the upper mold 20 and the polymer 12, and not to pass through the lower mold 30. Accordingly, during machining, upper and lower surfaces of the polymer 12 are set in a state of being covered with the mold 100, thus making it possible to prevent adhesion of a cutting powder or dust generated during machining, to the front optical surface 12a and the rear optical surface 12b.

When a thickness of the mold 100 is relatively large, the thickness of at least either one of the upper mold 20 and the lower mold 30 of the mold 100 may be machined to be thin (to the degree of not reaching the polymer 12 pinched between the upper and lower molds 20, 30) by a lathe, etc, prior to lens shape machining. As a mechanical cutting process, a method that is ordinarily performed by a skilled person is adopted. However, ordinarily, a foldable soft intraocular lens material is soft at a normal temperature, and therefore, when such a lens material is hardly machined at a normal temperature, it can be molded into a desired intraocular lens shape by cooling and cutting a copolymer. Further, even in a case that the lens material does not have hardness sufficient to be gripped by a cutting machine, although the material itself can be mechanically cut by cooling and cutting, it can be machined without applying complicated pre-treatment, etc, by polymerizing the material in the plastic mold that can be gripped by the cutting machine and machining it together with the mold.

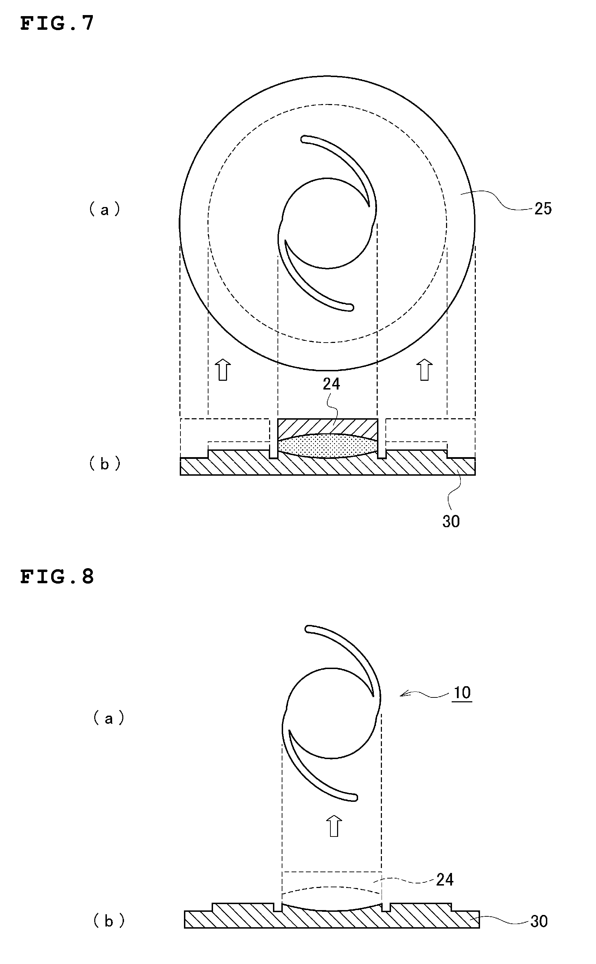

After the discoid polymer 12 is machined by the endmill 70 together with the mold 100, cut pieces of the upper mold 20 and the lower mold 30 are removed and the intraocular lens 10 that has undergone machining is taken out. FIG. 7 is a view showing a state that a cut piece 25 after the discoid polymer 12 is machined by the endmill 70 together with the mold 100, is separated and removed, wherein FIG. 7(a) is a plan view and FIG. 7(b) is a cross-sectional view. FIG. 8 is a view showing a state that the intraocular lens 10 is peeled off from the lower mold 30 together with a cut piece 24 after the discoid polymer 12 is machined by the endmill 70 together with the mold 100, wherein FIG. 8(a) is a view showing the peeled intraocular lens 10, and FIG. 8(b) is a cross-sectional view of the lower mold 30 after the intraocular lens 10 is peeled off.

As shown in FIG. 7, the cut piece 25 forming a part of the upper mold 30 is removed, and next, as shown in FIG. 8, the cut piece 24 forming a part of the upper mold 30 is peeled off from the intraocular lens 10, and subsequently, the intraocular lens 10 is peeled off from the lower mold 30. As a method of peeling off the intraocular lens 10 from the upper mold 20, etc, a lens may be peeled off by physically directly gripping it. However, one surface of the lens is exposed by removing the cut piece of the mold covering the lens, and thereafter the mold (lower mold 30) with the lens yet stuck thereto may be mechanically pressed from the side with no lens stuck thereto. Thus, the lens can be taken out without being damaged.

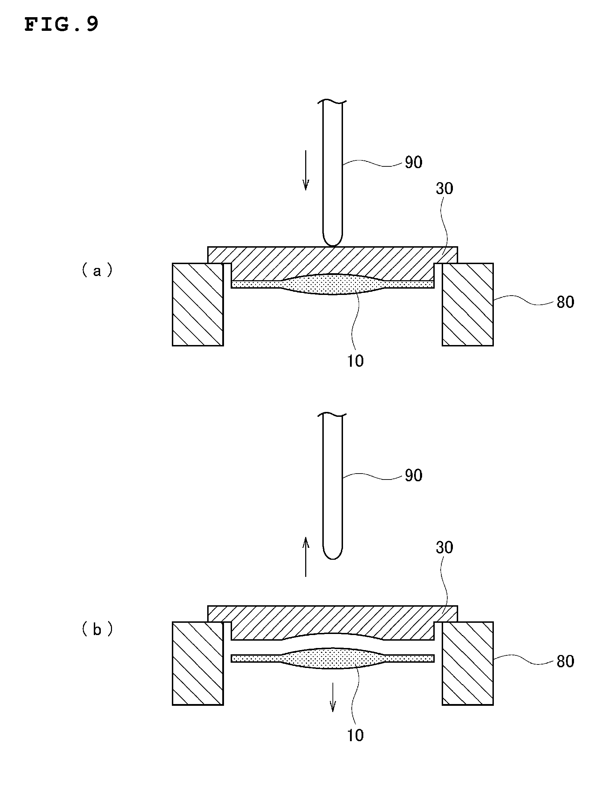

FIG. 9 is a view showing an example of a method of peeling off the intraocular lens stuck to the lower mold 30, wherein FIG. 9(a) is a view showing a state that the lower mold 30 is fixed to a fixing jig 80 with the intraocular lens 10 faced downward, and the lower mold 30 is pressed downward by a pressing rod 90 from the rear side, and FIG. 9(b) is a view showing a state that the intraocular lens 10 is peeled off. Thus, by the method shown in FIG. 9, the intraocular lens 10 can be taken out without being damaged. At this time, it is effective to cool the lens together with the mold and harden the lens moderately, for improving the release of the lens from the mold.

Further, when the cut piece of the mold covering the lens is removed, only the mold cut piece is peeled off from the lens and the lens is stuck to one of the upper and lower molds. In order to secure this state, one of the molds to which the lens is supposed to be stuck, may be strongly treated before injecting the raw material monomer, even when adhesive strength imparting treatment is applied only to one of the molds of upper and lower molds to which the lens is stuck, or the adhesive strength imparting treatment is applied to both upper and lower molds. Further, it is also preferable to use a method of peeling off the lens from the mold by putting it into water or alcohol so as to be swelled therein, because the lens can be taken out without being damaged. In the obtained intraocular lens 10, an advanced optical design having high accuracy, with mold inner surface transferred thereto, is given to the optical surface, and the optical surface has no cut powder or dust adhered thereto, which is generated during shape machining. Thus the intraocular lens 10 is a completed product with no polishing required.

Hereafter is shown examples of manufacturing the intraocular lens 10 by the manufacturing method described in the aforementioned embodiments. However, the present invention is not limited thereto. Of course as the mold, etc, used in the examples given hereafter, the aforementioned mold 100 is used.

### <Example 1>

65 pts.wt. of 2-phenyl ethyl acrylate, 30 pts.wt. of 2-pheyl ethyl methacrylate, 3.2 pts.wt. of butanediol diacrylate as a crosslinking monomer, and 0.3 pts.wt. of 2,2'azobis (isobutyronitrile) as a polymerization initiator, were prepared in a sample tube, which were then stirred sufficiently, to thereby obtain a homogeneous monomer mixed solution. The mixed solution was injected into one set of upper and lower polypropylene molds having the optical surface on the inner surface, and polymerization was performed in a pressure polymerizer based on a predetermined temperature program in a nitrogen atmosphere under pressure of 0.2MPa. Namely, the temperature was increased up to 50°C for 30 minutes from a room temperature, the mixed solution was held for 12 hours, the temperature was increased up to 100°C for 300 minutes, then the temperature was continuously increased up to 120° for 60 minutes, and the mixed solution was held for 2 hours, and the temperature was decreased. Thus, the soft intraocular lens material was polymerized.

Next, the obtained polymer was cut out into a lens shape by mechanical milling, without taking it out from the mold but in a state of being pinched between the molds. At this time, the endmill digs into the mold so as to pass through the upper side mold and the polymer and not to pass through the lower side mold, at a depth of forming a slight slit, and machining was performed while spraying a cool air set at -20°C. In addition, before lens shape machining, the upper side mold was machined to be thin in a machining direction of the endmill by using a lathe, so as not to reach the polymer.

After lens shape machining, the cut piece of the mold covering one surface of the lens was removed, in such a manner that one surface is exposed and one surface is in a state of being stuck to the mold. Thereafter, the lens was put in a refrigerator and cooled at 10°C together with the mold, to thereby slightly harden the lens. Subsequently, the mold with the lens yet stuck thereto, was mechanically pressed from the side with the lens not stuck thereto, to thereby remove the lens from the mold. No adhesion of the cut powder or dust generated during shape machining, was recognized in the obtained lens, and the soft intraocular lens requiring no polishing, and having the optical surface with the mold inner surface transferred thereto, was obtained.

### <Example 2>

In the same way as the example 1 other than a point that the adhesive strength imparting treatment was previously applied to the inner surface of one of the upper and lower sets of polypropylene molds used in the example 1 (the mold becoming the lower side of the endmill during lens shape machining) for 10 seconds in the air by the low pressure mercury lamp (produced by SEN LIGHTS CORPORATION "OPTICAL SURFACE TREATMENT APPARATUS PL16-110"), the soft intraocular lens was obtained. When the cut piece of the mold covering one surface of the lens was removed after lens shape machining, only the mold cut piece was peeled off from the lens, and the lens was in a state of being surely stuck to the other mold to which the adhesive strength imparting treatment was applied. No adhesion of the cut powder or dust generated during shape machining was recognized in the obtained lens, and the soft intraocular lens having the optical surface to which the mold inner surface was transferred, and requiring no polishing could be obtained.

### <Example 3>

In the same way as the example 1 other than a point that the monomer mixed solution was used as the raw material, which had a compositional ratio of 72 pts.wt. of 2-phenyl ethyl acrylate, 3 pts.wt. of methyl methacrylate, 5 pts.wt. of trifluoro ethyl methacrylate, 20 pts.wt. of 1,4-bis(2-hydroxyethoxy)phenylacrylate, 4.5 pts.wt. of neopentyl glycol diacrylate as a cross-linking monomer, 1.5 pts.wt. of 2-(2-hydroxy-3-tert-butyl-5-methyl phenyl)-5-(2-methacryloyloxyethyl) benzotriazole as a UV-absorbing component, 0.03 pts.wt. of 4-(5-hydroxy-3-methyl-1-phenyl-4-pyrazolyl methylene)-3-methacrylamino-1-phenyl-2-pyrazoline-5-on as a yellow coloring component, and 0.3 pts.wt. of 2,2'-azobis (isobutyronitrile) as a polymerization initiator, the soft intraocular lens was obtained. No adhesion of the cut powder or dust generated during shape machining was recognized in the obtained lens, and the soft intraocular lens having the optical surface to which the mold inner surface was transferred, and requiring no polishing could be obtained.

### <Example 4>

In the same way as the example 2 other than a point that the monomer mixed solution was used as the raw material, which had a compositional ratio of 56 pts.wt. of 2-phenyl ethyl methacrylate, 35 pts.wt. of n-butyl acrylate, 90 pts.wt. of 2-[2-(perfluorooctyl)ethoxy]-1-methyl ethyl methacrylate, 3 pts.wt. of ethylene glycol dimethacrylate as the cross-linking monomer, 0.3 pts.wt. of 2,2'-azobis (isobutyronitrile) as a polymerization initiator, the soft intraocular lens was obtained. No adhesion of the cut powder or dust generated during shape machining was recognized in the obtained lens, and the soft intraocular lens having the optical surface to which the mold inner surface was transferred, and requiring no polishing could be obtained.

### <Comparative example 1>

The soft intraocular lens material was polymerized in the same way as the example 1, and thereafter the upper and lower molds were disassembled, then one surface of the discoid polymer was exposed, and the other surface was set in a state of being stuck to the mold. Then, the endmill digs into the mold so as to pass through the upper side mold and the polymer and not to pass through the lower side mold, at a depth of forming a slight slit on the mold, and machining was performed while spraying a cool air set at -20°C, and the polymer was cut out into a lens shape by mechanical milling. After lens shape machining, the lens was put in a refrigerator and cooled at 10°C together with the mold, in a state that one surface was stuck to the mold, to thereby slightly harden the lens. Subsequently, the mold to which the lens was yet stuck, was mechanically pressed from the side with no lens stuck thereto, to thereby take out the lens from the mold. In the obtained lens, a lot of cut powders or dusts generated during machining were adhered to the surface exposed during shape machining, and the lens of an acceptable level in outer appearance test could not be obtained. Further, subsequently, tumbling polishing was applied to the obtained lens for 72 hours, by using glass beads with particle diameter of 0.8mm. However, the adhered cut powders could not be completely removed.

### <Comparative example 2>

After the soft intraocular lens material was polymerized in the same way as the example 3, the soft intraocular lens was obtained in the same way as the comparative example 1. In the obtained lens, a lot of cut powders or dusts generated during machining were adhered to the surface exposed during shape machining, and the lens of an acceptable level in outer appearance test could not be obtained. Further, subsequently, tumbling polishing was applied to the obtained lens for 72 hours, by using glass beads with particle diameter of 0.8mm. However, the adhered cut powders could not be completely removed.

### <Comparative example 3>

After the soft intraocular lens material was polymerized in the same way as the example 4, the soft intraocular lens was obtained in the same way as the comparative example 1. In the obtained lens, a lot of cut powders or dusts generated during machining were adhered to the surface exposed during shape machining, and the lens of an acceptable level in outer appearance test could not be obtained. Further, subsequently, tumbling polishing was applied to the obtained lens for 72 hours, by using glass beads with particle diameter of 0.8mm. However, the adhered cut powders could not be completely removed.

### Industrial Applicability

The present invention can be utilized for manufacturing an intraocular lens inserted into an eye, instead of a crystalline lens which is removed when the crystalline lens is getting cloudy due to a cataract, etc.

### Brief description of the drawings

FIG. 1 is a view showing an intraocular lens manufactured by a manufacturing method of the intraocular lens according to an embodiment of the present invention, wherein FIG. 1(a) is a plan view and FIG. 1(b) is a side view.
FIG. 2 is a view showing a mold used for the manufacturing method of the intraocular lens according to the embodiment of the present invention, wherein FIG. 2(a) is a cross-sectional view showing a state that an upper mold and a lower mold are separated from each other, and FIG. 2(b) is a cross-sectional view showing a state that the upper mold and the lower mold are connected to each other in a state of being molded, and FIG. 2(c) is a plan view.
FIG. 3 is a view showing a state that a raw material is injected into a mold 100 and molding is performed.
FIG. 4 is a cross-sectional view showing a state that a discoid polymer 12 is fixed to a fixing jig 60 and is machined by an endmill 70 together with the mold 100.

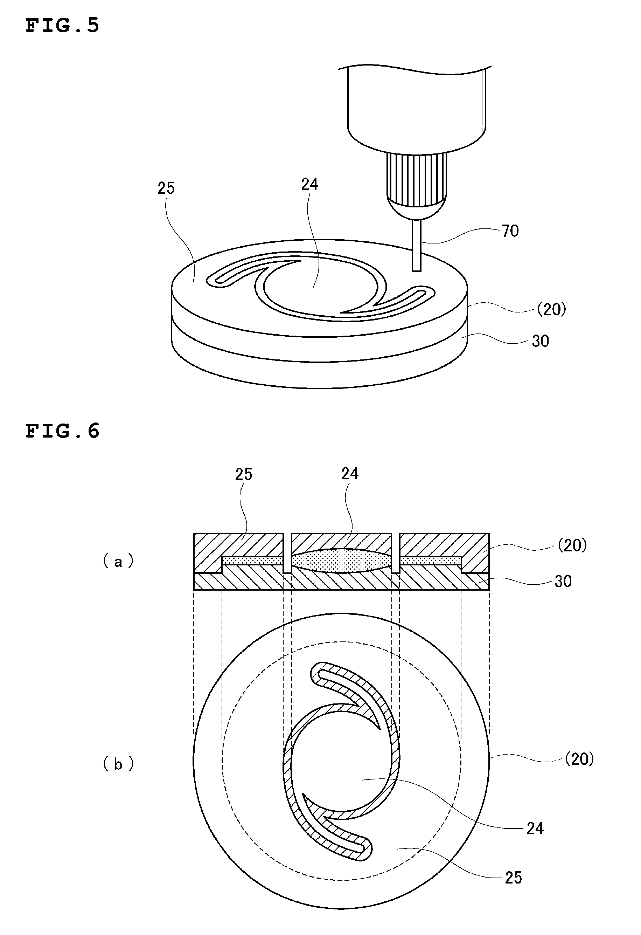

FIG. 5 is a perspective view showing a state that the discoid polymer 12 is machined by the endmill 70 together with the mold 100.
FIG. 6 is a view showing a state after the discoid polymer 12 is machined by the endmill 70 together with the mold 100, wherein FIG. 6 (a) is a cross-sectional view and FIG. 6(b) is a plan view.
FIG. 7 is a view showing a state that a cut piece 25 is removed after the discoid polymer 12 is machined by the endmill 70 together with the mold 100, wherein FIG. 7 (a) is a plan view and FIG. 7(b) is a cross-sectional view.
FIG. 8 is a view showing a state that an intraocular lens 10 is peeled off from a lower mold 30 after the discoid polymer 12 is machined by the endmill 70 together with the mold 100, wherein FIG. 8(a) is a view showing the peeled intraocular lens 10 and FIG. 8(b) is a cross-sectional view of the lower mold 30 after the intraocular lens 10 is peeled off.
FIG. 9 is a view showing an example of a method of peeling the intraocular lens stuck to the lower mold 30, wherein FIG. 9 (a) is a view showing a state that the lower mold 30 is fixed to the fixing jig 80 with the intraocular lens 10 faced downward, and the lower mold 30 is pressed downward by a pressing rod 90 from the rear side, and FIG. 9(b) is a view showing a state that the intraocular lens 10 is peeled off.

### Description of Signs and Numerals

- 10: Intraocular lens
- 11: Optical part
- 11a: Front optical surface
- 11b: Rear optical surface
- 12: Support part precursor member
- 12a, 12b: Support part
- 20: Upper mold
- 30: Lower mold
- 100: Mold

## Claims

1. A manufacturing method of an intraocular lens (10) comprising an optical part (11) having a front optical surface (11a) and a rear optical surface (11b) that constitute front and rear surfaces of a lens; and a support part (12a, 12b) provided on an outer peripheral part of the optical part (11) for supporting the optical part (11) in an eye,
wherein a mold (100) is used, having an optical part molding part for forming a member becoming the optical part by molding, and a support part molding part for forming a member (12) becoming the support part by molding, wherein the front optical surface (11a) and the rear optical surface (11b) can be directly obtained by molding the optical molding part, and the support part (12a, 12b) can be obtained by applying a additional machining to the member formed by molding,
the method comprising the steps of:
injecting a raw material of an intraocular lens into the mold, then polymerizing or hardening the raw material, then
machining the member becoming the support part into a shape of the support part in a state that at least the front optical surface and the rear optical surface in the polymerized or hardened members are covered with the optical part molding part of the mold, and
releasing the optical part molding part from the mold.

2. The manufacturing method of the intraocular lens according to claim 1,
wherein the support part molding part of the mold has a sectional face with a shape of a completed support part if cut by a sectional face including an optical axis of the optical part, and with an arbitrary shape if viewed from the optical axis direction, and machining by the support part molding part is applied to the member becoming the support part so that the shape of the member becoming the support part viewed from the optical axis direction corresponds to the shape of the completed support part.

3. The manufacturing method of the intraocular lens according to claim 1 or 2, wherein a raw material of the intraocular lens is injected into the mold, which is then polymerized or hardened, and in a state that polymerized or hardened members exist in both the optical part molding part and the support part molding part of the mold, the members are machined together with the mold so that the shape viewed from the optical axis direction in the member becoming the optical part and the member becoming the support part corresponds to the shape of the completed intraocular lens.

4. The manufacturing method of the intraocular lens according to any one of claims 1 to 3, wherein the mold has an upper mold and a lower mold,
the method comprising the steps of:
injecting the raw material of the intraocular lens into the mold, then polymerizing or hardening the raw material; then
machining the member becoming the optical part and/or the member becoming the support part together with the mold in a state that the members exist between the upper mold and the lower mold, so that the shape viewed from the optical axis direction corresponds to a completed shape,
wherein in the machining step, machining is applied to the mold with the members existing inside in such a manner as digging into one of the upper mold and the lower mold of the mold from the optical axis direction along a virtual contour line that partitions so that the shape viewed from the optical axis direction corresponds to the completed shape to a depth of passing through one of the molds and to a prescribed depth of the other mold, over an entire circumference of the contour line.

5. The manufacturing method of the intraocular lens according to any one of claims 1 to 4, wherein the intraocular lens is a soft intraocular lens.

6. The manufacturing method of the intraocular lens according to any one of claims 1 to 5, wherein the mold is composed of plastic resin.

7. The manufacturing method of the intraocular lens according to claim 6, wherein the plastic resin is polyolefin resin, polyethylene resin, or polypropylene resin.

8. The manufacturing method of the intraocular lens according to claim 6, wherein a glass transition temperature of a material composing the soft intraocular lens is less than 35°C.

9. The manufacturing method of the intraocular lens according to claim 6, wherein a material composing the soft intraocular lens is a polymer of acrylic monomer.

10. The manufacturing method of the intraocular lens according to claim 6, wherein a treatment for imparting adhesive strength to the material composing the optical part is previously applied to an inner surface of the mold made of plastic resin.

11. The manufacturing method of the intraocular lens according to claim 10, wherein in the treatment for imparting adhesive strength to the material, an active light, which has its emission peak at 150-300 nm wavelength range and has a function that decomposes an oxygen molecule to generate ozone and decomposes the ozone to generate active oxygen species, is previously irradiated to the first polymer under existence of oxygen.

## Patentansprüche

1. Verfahren zum Herstellen einer Intraokularlinse (10) mit einem Optikteil (11), der eine vordere Optikfläche (11a) und eine hintere Optikfläche (11b) besitzt, die eine vordere und eine hintere Oberfläche der Linse darstellen; und einem Trägerteil (12a, 12b), der auf einem äußeren Umfangsteil des Optikteils (11) zum Tragen des Optikteils (11) in einem Auge vorgesehen ist,
wobei eine Form (100) verwendet wird, die einen Optikteil-Formteil zur Bildung eines Elements, das zu dem Optikteil wird, durch Gießen besitzt und ein Trägerteil-Formteil zur Bildung eines Elements (12), das zu dem Trägerteil wird, durch Gießen besitzt, wobei die vordere Optikfläche (11a) und die hintere Optikfläche (11b) direkt durch Gießen des Optik-Formteils erhalten werden können, und der Trägerteil (12a, 12b) durch Anwenden einer zusätzlichen Verarbeitung auf das durch Gießen gebildete Element erhalten werden kann,
wobei das Verfahren die folgenden Schritte umfasst:
Einspritzen eines Rohmaterials einer Intraokularlinse in die Form, dann Polymerisieren oder Härten des Rohmaterials, dann
Verarbeiten des Elements, das zu dem Trägerteil wird, in eine Gestalt des Trägerteils in einem Zustand, in dem zumindest die vordere Optikfläche und die hintere Optikfläche in den polymerisierten oder gehärteten Elementen mit dem Optikteil-Formteil der Form bedeckt sind, und
Lösen des Optikteil-Formteils aus der Form.

2. Verfahren zum Herstellen der Intraokularlinse nach Anspruch 1,
wobei der Trägerteil-Formteil der Form eine Schnittfläche mit einer Gestalt eines fertiggestellten Trägerteils besitzt, wenn er von einer Schnittfläche geschnitten wird, die eine optische Achse des Optikteils umfasst, und eine Schnittfläche mit einer beliebigen Gestalt besitzt, wenn er aus der Richtung der optischen Achse betrachtet wird, und ein Verarbeiten durch den Trägerteil-Formteil auf das Element angewendet wird, das zu dem Trägerteil wird, so dass die Gestalt des Elements, das zu dem Trägerteil wird, wenn sie aus der Richtung der optischen Achse betrachtet wird, der Gestalt des fertiggestellten Trägerteils entspricht.

3. Verfahren zum Herstellen der Intraokularlinse nach Anspruch 1 oder 2, wobei ein Rohmaterial der Intraokularlinse in die Form eingespritzt wird, das dann polymerisiert oder gehärtet wird, und in einem Zustand, in dem polymerisierte oder gehärtete Elemente sowohl in dem Optikteil-Formteil als auch in dem Trägerteil-Formteil der Form vorhanden sind, die Elemente zusammen mit der Form so verarbeitet werden, dass die Gestalt aus der Richtung der optischen Achse gesehen in dem Element, das zu dem Optikteil wird, und dem Element, das zu dem Trägerteil wird, der Gestalt der fertiggestellten Intraokularlinse entspricht.

4. Verfahren zum Herstellen der Intraokularlinse nach einem der Ansprüche 1 bis 3, wobei die Form eine obere Form und eine untere Form umfasst,
wobei das Verfahren die folgenden Schritte umfasst:
Einspritzen eines Rohmaterials einer Intraokularlinse in die Form, dann Polymerisieren oder Härten des Rohmaterials; dann
Verarbeiten des Elements, das zu dem Optikteil wird, und/oder des Elements, das zu dem Trägerteil wird, zusammen mit der Form in einem Zustand, in dem die Elemente zwischen der oberen Form und der unteren Form vorhanden sind, so dass die Gestalt, wenn sie in Richtung der optischen Achse betrachtet wird, einer fertiggestellten Gestalt entspricht,
wobei in dem Schritt des Verarbeitens das Verarbeiten auf die Form, innerhalb der die Elemente vorhanden sind, in der Weise angewendet wird, dass in die obere Form oder die untere Form der Form aus der Richtung der optischen Achse entlang einer virtuellen Konturlinie gegraben wird, die so unterteilt, dass die Gestalt, wenn sie in Richtung der optischen Achse betrachtet wird, bis zu einer Tiefe des Passierens durch eine der Formen und bis zu einer vorgeschriebenen Tiefe der anderen Form über einen gesamten Umfang der Konturlinie der fertiggestellten Gestalt entspricht.

5. Verfahren zum Herstellen der Intraokularlinse nach einem der Ansprüche 1 bis 4, wobei die Intraokularlinse eine weiche Intraokularlinse ist.

6. Verfahren zum Herstellen der Intraokularlinse nach einem der Ansprüche 1 bis 5, wobei die Form aus Kunststoffharz besteht.

7. Verfahren zum Herstellen der Intraokularlinse nach Anspruch 6, wobei das Kunststoffharz ein Polyolefinharz, ein Polyethylenharz oder ein Polypropylenharz ist.

8. Verfahren zum Herstellen der Intraokularlinse nach Anspruch 6, wobei eine Glasübergangstemperatur eines Materials, aus dem die weiche Intraokularlinse besteht, niedriger als 35 °C ist.

9. Verfahren zum Herstellen der Intraokularlinse nach Anspruch 6, wobei ein Material, aus dem die weiche Intraokularlinse besteht, ein Polymer aus einem Acrylmonomer ist.

10. Verfahren zum Herstellen der Intraokularlinse nach Anspruch 6, wobei eine Behandlung zum Vermitteln von Haftfestigkeit an das Material, aus dem der Optikteil besteht, vorher auf eine Innenfläche der Form, die aus Kunststoffharz hergestellt wird, angewendet wird.

11. Verfahren zum Herstellen der Intraokularlinse nach Anspruch 10, wobei bei der Behandlung zum Vermitteln von Haftfestigkeit an das Material aktives Licht, das ein Emissionsmaximum in einem Wellenlängenbereich von 150-300 nm besitzt und die Funktion hat, ein Sauerstoff-Molekül zu zersetzen, um Ozon zu erzeugen, und das Ozon zu zersetzen, um aktive Sauerstoffspezies zu erzeugen, zuvor unter Vorhandensein von Sauerstoff auf das erste Polymer eingestrahlt wird.

## Revendications

1. Procédé de fabrication d'une lentille intraoculaire (10) comprenant une partie optique (11) ayant une surface optique avant (11a) et une surface optique arrière (11b) qui constituent les surfaces avant et arrière d'une lentille ; et une partie de support (12a, 12b) prévue sur une partie périphérique extérieure de la partie optique (11) pour supporter la partie optique (11) dans un oeil,
dans lequel on utilise un moule (100) ayant une partie de moulage de partie optique pour former un élément qui devient la partie optique par moulage, et une partie de moulage de partie de support pour former un élément (12) qui devient la partie de support par moulage,
dans lequel la surface optique avant (11a) et la partie optique arrière (11b) peuvent être directement obtenues en moulant la partie optique moulée, et la partie de support (12a, 12b) peut être obtenue en appliquant un usinage additionnel à l'élément formé par moulage, le procédé comprenant les étapes consistant à :
injecter un matériau brut d'une lentille intraoculaire dans le moule, puis polymériser ou durcir le matériau brut, puis usiner l'élément qui devient la partie optique sous la forme de la partie optique dans un état dans lequel au moins la surface optique avant et la surface optique arrière dans les éléments polymérisés ou durcis sont couvertes avec la partie de moulage de partie optique du moule, et libérer la partie de moulage de partie optique hors du moule.

2. Procédé de fabrication de lentille intraoculaire selon la revendication 1,
dans lequel la partie de moulage de partie de support du moule comporte une face en section avec une forme d'une partie de support terminée si elle est coupée par une face en section incluant un axe optique de la partie optique, et avec une forme arbitraire si on la voit depuis la direction de l'axe optique, et l'usinage par la partie de moulage de partie de support est appliqué à l'élément qui devient la partie de support de telle façon que la forme de l'élément qui devient la partie de support vue depuis la direction de l'axe optique correspond à la forme de la partie de support terminée.

3. Procédé de fabrication de lentille intraoculaire selon la revendication 1 ou 2, dans lequel un matériau brut de la lentille intraoculaire est injecté dans le moule, et est ensuite polymérisé ou durci, et dans un état dans lequel des éléments polymérisés ou durcis existent à la fois dans la partie de moulage de partie optique et dans la partie de moulage de partie de support du moule, les éléments sont usinés ensemble avec le moule de telle façon que la forme, vue depuis la direction de l'axe optique dans l'élément qui devient la partie optique et l'élément qui devient la partie de support, correspond à la forme de la lentille intraoculaire terminée.

4. Procédé de fabrication de lentille intraoculaire selon l'une quelconque des revendications 1 à 3, dans lequel le moule comprend un moule supérieur et un moule inférieur,
le procédé comprenant les étapes consistant à :
injecter le matériau brut de la lentille intraoculaire dans le moule, puis polymériser ou durcir le matériau brut ; puis usiner l'élément qui devient la partie optique et/ou l'élément qui devient la partie de support ensemble avec le moule dans un état dans lequel les éléments existent entre le moule supérieur et le moule inférieur, de sorte que la forme, vue depuis la direction de l'axe optique, correspond à une forme terminée,
dans lequel, dans l'étape d'usinage, l'usinage est appliqué au moule avec les éléments existants à l'intérieur de manière à creuser dans un moule parmi le moule supérieur et le moule inférieur du moule depuis la direction de l'axe optique le long d'une ligne de contour virtuelle qui effectue un cloisonnement, de telle façon que la forme, vue depuis la direction de l'axe optique, correspond à la forme terminée à une profondeur passant à travers l'un des moules et jusqu'à une profondeur prescrite dans l'autre moule, sur une circonférence entière de la ligne de contour.

5. Procédé de fabrication de lentille intraoculaire selon l'une quelconque des revendications 1 à 4, dans lequel la lentille intraoculaire est une lentille intraoculaire souple.

6. Procédé de fabrication de lentille intraoculaire selon l'une quelconque des revendications 1 à 5, dans lequel le moule est composé de résine de matière plastique.

7. Procédé de fabrication de lentille intraoculaire selon la revendication 6, dans lequel la résine de matière plastique est une résine polyoléfine, une résine polyéthylène ou une résine polypropylène.

8. Procédé de fabrication de lentille intraoculaire selon la revendication 6, dans lequel une température de transition vitreuse d'un matériau qui compose la lentille intraoculaire souple est inférieure à 35° C.

9. Procédé de fabrication de lentille intraoculaire selon la revendication 6, dans lequel un matériau qui compose la lentille intraoculaire souple est un polymère de monomère acrylique.

10. Procédé de fabrication de lentille intraoculaire selon la revendication 6, dans lequel un traitement pour conférer une résistance adhésive au matériau composant la partie optique est auparavant appliqué à une surface intérieure du moule fait en résine de matière plastique.

11. Procédé de fabrication de lentille intraoculaire selon la revendication 10, dans lequel, dans le traitement pour conférer une résistance adhésive au matériau, une lumière active qui présente sa pointe d'émission à des longueurs d'onde dans la plage de 150 à 300 nm et dont la fonction est de décomposer une molécule d'oxygène pour générer de l'ozone et de décomposer l'ozone pour générer de l'oxygène actif, est auparavant irradiée sur le premier polymère en présence d'oxygéne.
